# EUROPÄISCHE PATENTSCHRIFT

(11) **EP 0 308 744 B1**
(45) Veröffentlichungstag und Bekanntmachung des Hinweises auf die Patenterteilung: **10.02.1993**
(21) Anmeldenummer: 88114752.4
(22) Anmeldetag: 09.09.1988
(51) Int. Cl.: C07D 233/32, C07D 233/34, C07D 263/22, C25B 3/02

(54) **Verfahren zur Herstellung von Imidazolidinonen und Oxazolidinonen**
Process for the preparation of imidazolidinones and oxazolidinones
Procédé de préparation d'imidazolidinones et oxazolidinones

(30) Priorität: 12.09.1987 DE 3730777
(43) Veröffentlichungstag der Anmeldung: 29.03.1989
(73) Patentinhaber: BASF Aktiengesellschaft, 67063 Ludwigshafen (DE)
(72) Erfinder: Degner, Dieter, Dr., D-6701 Dannstadt-Schauernheim (DE)

(56) Entgegenhaltungen:
- EP-A- 0 125 986
- EP-A- 0 192 931
- EP-A- 0 212 512
- EP-A- 0 237 762
- TETRAHEDRON, Band 32, Nr. 18, 1. Oktober 1976, Seiten 2185-2206, Pergamon Press, Dublin, IE; L. EBERSON et al.: "Synthetic uses of anodic substitution reactions"

## Beschreibung

Die vorliegende Erfindung betrifft ein neues elektrochemisches Verfahren für die Herstellung von Imidazolidinonen und Oxazolidinonen.

Imidazolidinon-2 wird z. B. aus Glykol, Ethylendiamin oder 2-Aminoethanol-1 durch Umsetzung mit Harnstoff hergestellt. Dabei werden jedoch hohe Temperaturen (über 200°C) benötigt (J. Org. Chem. 15, 475-480 [1950]). Oxazolidinon-2 wird z.B. durch Umsetzung von 2-Aminoethanol-1 mit dem Anlagerungsprodukt von Phosgen an Pyridin erhalten (Chem. Ber. 89, 2565 [1956]). Bei dieser Synthese sind die Verwendung hochtoxischer Stoffe sowie der Anfall von Abfallsalzen von Nachteil.

Aus Tetrahedron 32, 2185 - 2206 [1976] ist bekannt, daß Amide, wie Formamide elektrochemisch zu Alkoxiformamiden oxidiert werden. In der europäischen Patentanmeldung 212 512 wird ein Verfahren zur Herstellung von Carbamidsäureestern durch Elektrooxidation von Formamiden beschrieben.

Es wurde nun überraschenderweise gefunden, daß man Imidazolidinone und Oxazolidinone der allgemeinen Formel 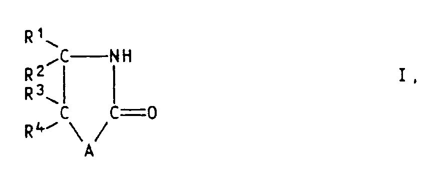
in der A für -O- oder -NH- steht und R¹ R² R³ und R⁴ Wasserstoffatome, Alkylgruppen mit 1 bis 6 C-Atomen oder Phenylgruppen bedeuten, besonders vorteilhaft dadurch herstellen kann, daß man Formamide der allgemeinen Formel 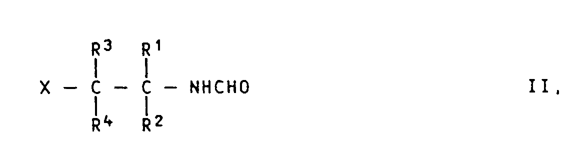
in der X für -OH oder -NHCHO steht und die Reste R¹ bis R⁴ die obengenannte Bedeutung haben, in einem Alkanol als Lösungsmittel in Gegenwart eines ionogenen Halogenids elektrochemisch oxidiert.

Die Ausgangsstoffe der Formel II sind Formamide, die sich von 1,2-Diaminoethan und 1-Amino-2-hydroxiethan ableiten und die als Substituenten R¹ bis R⁴ Alkylgruppen mit 1 bis 6 C-Atomen, wie Methyl, Ethyl, Propyl, iso-Propyl, Butyl, iso-Butyl, tert.-Butyl oder Phenylgruppen enthalten können. Beispielsweise seien die Ausgangsstoffe der Formeln
OHCHN-CH₂-CH₂-NHCHO, HO-CH₂-CH₂-NHCHO und 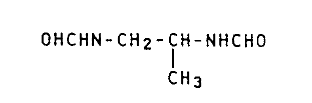
genannt.

Als ionogene Halogenide kommen Salze, wie die Alkali-, Erdalkali- oder Ammoniumsalze der Jodwasserstoff-, Bromwasserstoff- und Chlorwasserstoffsäure in Betracht. Besonders bevorzugt sind Salze der Bromwasserstoffsäure, wie Alkali- und Erdalkalibromide sowie quartäre Ammonium- - insbesondere Tetraalkylammoniumbromide. Das Kation spielt keine erfindungswesentliche Rolle. Zweckmäßigerweise wird man billige Halogenide wählen. Beispielsweise seien Lithium-, Natrium-, Calcium-, Ammonium- sowie Tetramethylammoniumbromid genannt.

Das erfindungsgemäße Verfahren erfordert keine besondere Elektrolysezelle. Vorteilhaft kann man es in einer ungeteilten Durchflußzelle durchführen. Als Anoden können alle an sich üblichen Anodenmaterialien verwendet werden, die unter den Elektrolysebedingungen stabil sind, wie Edelmetalle, z.B. Platin. Bevorzugtes Anodenmaterial ist Graphit. Das Kathodenmaterial besteht z. B. aus Metallen wie Blei, Eisen, Stahl, Nickel oder Edelmetallen, wie Platin. Bevorzugtes Kathodenmaterial ist ebenfalls Graphit.

Man führt die elektrochemische Oxidation so durch, daß man die Ausgangsstoffe, als Lösung in einem geeigneten Lösungsmittel elektrolysiert. Geeignete Lösungsmittel sind Alkanole, wie Methanol, Ethanol, Propanol, Isopropanol und Butanole, von denen Methanol bevorzugt ist. Der Elektrolyt besteht somit bevorzugt aus einer Lösung der Ausgangsstoffe, die ein ionogenes Halogenid enthält. Seine Zusammensetzung kann in weiten Grenzen gewählt werden. Beispielsweise hat der Elektrolyt die folgende Zusammensetzung:
1 bis 50 Gew.% Formamid der Formel II
40 bis 90 Gew.% Lösungsmittel
0,1 bis 10 Gew.% Halogenid.

Die Stromdichte ist für das erfindungsgemäße Verfahren kein begrenzter Faktor, sie beträgt z.B. 1 bis 25 A/dm², vorzugsweise wird mit einer Stromdichte von 3 bis 12 A/dm² elektrolysiert. Man elektrolysiert z.B. bei Temperaturen bis 120°C. Die Temperatur wird bei druckloser Fahrweise der Elektrolyse zweckmäßigerweise so gewählt, daß sie zumindest 5 bis 10°C unter dem Siedepunkt des Elektrolyten liegt. Bei Verwendung von Methanol oder Ethanol wird vorzugsweise bei Temperaturen von 20 bis 50°C elektrolysiert.

Das neue Verfahren erlaubt es, die Formamide vollständig umzusetzen, ohne daß es zu Ausbeuteverschlechterungen kommt.

Die Aufarbeitung der Elektrolyseausträge kann man nach an sich bekannten Methoden vornehmen. Zweckmäßigerweise wird aus dem Elektrolyseaustrag zunächst das Lösungsmittel abdestilliert. Die Halogenide werden dann, z.B. durch Filtration oder Extraktion abgetrennt. Die Heterocyclen fallen danach häufig in sehr reiner Form an, sie können bei Bedarf, z.B. durch Umfällen oder Umkristallisation weiter gereinigt werden. Lösungsmittel sowie Halogenide können zur Elektrolyse zurückgeführt werden. Das erfindungsgemäße Verfahren kann sowohl diskontinuierlich als auch kontinuierlich durchgeführt werden.

Die nach dem erfindungsgemäßen Verfahren hergestellten Heterocyclen sind vielfältig einsetzbare Zwischenprodukte, die z.B. als Vorprodukte für Textilhilfsmittel und Farbstoffe Verwendung finden.

### Beispiel 1

### Synthese von Imidazolidinon-2

- Apparatur :: ungeteilte Zelle mit 11 Graphitelektroden
- Anoden :: Graphit
- Elektrolyt :: 360 g (3,10 Mol) des Formamids der Formel OHCHN-(CH₂)₂-NHCHO
36 g NaBr
3204 g CH₃OH
- Kathoden :: Graphit
- Stromdichte:: 3,3 A/dm²
- Temperatur :: 25°C

Elektrolyse mit 3,1 F/Mol OHCNH-(CH₂)₂-NHCHO.

Der Elektrolyt wurde während der Elektrolyse mit 200 l/h durch die Zelle über einen Wärmetauscher gepumpt.

Aufarbeitung: Nach Beendigung der Elektrolyse wurde Methanol bei Normaldruck abdestilliert und der Rückstand (289 g) aus Ethanol-Toluol umkristallisiert. Es wurden 259 g Imidazolidinon-2 erhalten (Schmelzbereich: 132 bis 150°C, HNMR, C¹³-NMR-Spektren stimmen mit den Spektren überein, die an authentischer Substanz gemessen wurden). Dies entspricht einer Ausbeute von 94,8 %.

### Beispiel 2

### Synthese von 4-Methyl-imidazolidinon-2

- Apparatur :: ungeteilte Zelle mit 6 Elektroden
- Anoden :: Graphit
- Elektrolyt :: 260 g (2,0 Mol) der Verbindung der Formel 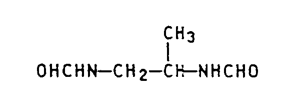 26 g NaBr
2314 g CH₃OH
- Kathoden :: Graphit
- Stromdichte:: 3,3 A/dm²
- Temperatur :: 27 - 28°C

Elektrolyse mit 4,5 F/Mol

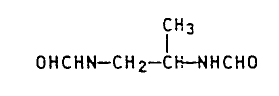

Der Elektrolyt wurde während der Elektrolyse mit 200 l/h durch die Zelle über einen Wärmetauscher gepumpt.

Aufarbeitung: Nach Beendigung der Elektrolyse wurde Methanol bei Normaldruck abdestilliert und der Rückstand aus Methylethylketon-Toluol umkristallisiert. Hierbei wurden 166 g 4-Methylimidazolidinon-2 erhalten (Fp. 112 bis 118°C, HNMR- und C¹³-NMR-spektrum entsprechen der vorgegebenen Struktur). Dies entspricht einer Ausbeute von 83 %.

### Beispiel 3

### Synthese von Oxazolidin-2-on

- Apparatur :: ungeteilte Zelle mit 11 Elektroden
- Anoden :: Graphit
- Elektrolyt :: 495 g (5,56 Mol) der Verbindung der Formel HO-CH₂-CH₂-NHCHO
33 g NaBr
3300 g CH₃OH
- Kathoden :: Graphit
- Stromdichte:: 3,3 A/dm²
- Temperatur :: 34°C

Elektrolyse mit 2,0 F/Mol HO-CH₂-CH₂NHCHO.
Der Elektrolyt wurde mit 200 l/h durch die Zelle über einen Wärmetauscher gepumpt.

Aufarbeitung: Nach Beendigung der Elektrolyse wurde Methanol bei Normaldruck abdestilliert und der Rückstand aus Ethanol-Petrolether umkristallisiert. Hierbei wurden 254 g Oxazolidin-2-on erhalten (Fp. 88 bis 89°C, HNMR- und C¹³NMR Spektrum entsprechen der angegebenen Struktur). Dies entspricht einer Ausbeute von 52.5 %.

## Patentansprüche

1. Verfahren zur Herstellung von Imidazolidinonen und Oxazolidinonen der allgemeinen Formel 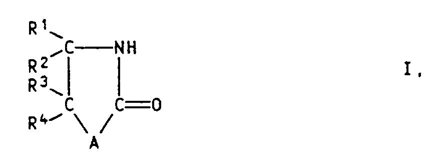 in der A für -O- oder -NH- steht und R¹, R², R³ und R⁴ Wasserstoffatome, Alkylgruppen mit 1 bis 6 C-Atomen oder Phenylgruppen bedeuten, dadurch gekennzeichnet, daß man Formamide der allgemeinen Formel 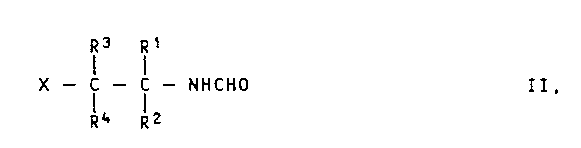 in der X für -OH oder -NHCHO steht und in einem Alkanol als Lösungsmittel in Gegenwart eines ionogenen Halogenids elektrochemisch oxidiert.

2. Verfahren nach Anspruch 1, dadurch gekennzeichnet, daß man die Elektrooxidation in Methanol durchführt.

3. Verfahren gemäß Anspruch 1, dadurch gekennzeichnet, daß man als ionogenes Halogenid ein Salz der Bromwasserstoffsäure verwendet.

4. Verfahren gemäß Anspruch 1, dadurch gekennzeichnet, daß man als Anoden Graphitanoden einsetzt.

5. Verfahren nach Anspruch 1, dadurch gekennzeichnet, daß man bei Stromdichten von 1 bis 25 A/dm² und Temperaturen bis 120°C elektrolysiert.

## Claims

1. A process for preparing imidazolidinones and oxazolidinones of the formula 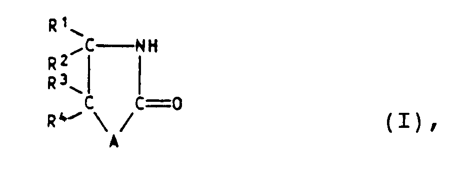 where A is -O- or -NH-, and R¹, R², R³ and R⁴ are each hydrogen, alkyl of 1-6 carbons or phenyl, which comprises electrochemical oxidation of formamides of the formula 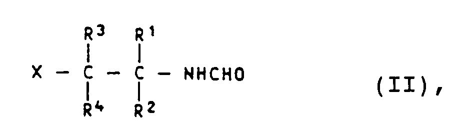 where X is -OH or -NHCHO, in an alkanol as solvent in the presence of an ionic halide.

2. A process as claimed in claim 1, wherein the electrooxidation is carried out in methanol.

3. A process as claimed in claim 1, wherein a salt of hydrobromic acid is used as ionic halide.

4. A process as claimed in claim 1, wherein graphite anodes are employed.

5. A process as claimed in claim 1, wherein electrolysis is carried out with current densities of 1-25 A/dm² at up to 120°C.

## Revendications

1. Procédé de préparation d'imidazolidinones et d'oxazolidinones de formule générale 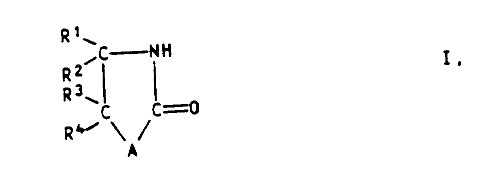 dans laquelle A est mis pour -O- ou -NH- et R¹, R², R³ et R⁴ représentent des atomes d'hydrogène, des groupements alkyle ayant de 1 à 6 atomes de carbone ou des groupements phényle, caractérisé en ce qu'on oxyde par voie électrochimique, dans un alcanol comme solvant et en présence d'un halogénure ionogène, des formamides de formule générale 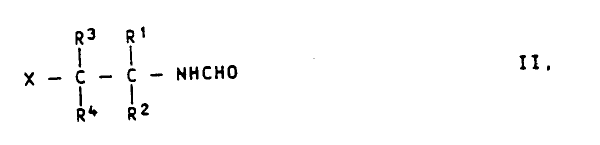 dans laquelle X représente -OH ou -NHCHO.

2. Procédé selon la revendication 1, caractérisé en ce qu'on effectue l'électrooxydation dans du méthanol.

3. Procédé selon la revendication 1, caractérisé en ce qu'on utilise comme halogénure ionogène un sel de l'acide bromhydrique.

4. Procédé selon la revendication 1, caractérisé en ce qu'on utilise comme anodes des anodes en graphite.

5. Procédé selon la revendication 1, caractérisé en ce qu'on électrolyse à des densités de courant de 1 à 25 A/dm² et à des températures allant jusqu'à 120°C.
